(19) Europäisches Patentamt / European Patent Office / Office européen des brevets

(11) **EP 1 965 761 B1**

(12) **EUROPÄISCHE PATENTSCHRIFT**

(45) Veröffentlichungstag und Bekanntmachung des Hinweises auf die Patenterteilung:
**01.07.2015 Patentblatt 2015/27**

(51) Int Cl.:
**A61K 9/00** *(2006.01)*    **A61K 9/20** *(2006.01)*

(21) Anmeldenummer: **06841327.7**

(22) Anmeldetag: **11.12.2006**

(86) Internationale Anmeldenummer:
**PCT/EP2006/069515**

(87) Internationale Veröffentlichungsnummer:
**WO 2007/071581 (28.06.2007 Gazette 2007/26)**

(54) **PHARMAZEUTISCHE FORMULIERUNG FÜR DIE HERSTELLUNG VON SCHNELL ZERFALLENDEN TABLETTEN**

PHARMACEUTICAL FORMULATION FOR PRODUCING RAPIDLY DISINTEGRATING TABLETS

FORMULATION PHARMACEUTIQUE DESTINEE A LA FABRICATION DE COMPRIMES A DESINTEGRATION RAPIDE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **21.12.2005 EP 05112654**
**23.12.2005 EP 05112969**

(43) Veröffentlichungstag der Anmeldung:
**10.09.2008 Patentblatt 2008/37**

(73) Patentinhaber: **BASF SE**
**67056 Ludwigshafen (DE)**

(72) Erfinder:
• **KOLTER, Karl**
**67117 Limburgerhof (DE)**
• **WICHTNER, Marcus**
**68163 Mannheim (DE)**
• **SCHÖNHERR, Michael**
**67227 Frankenthal (DE)**
• **MITTWOLLEN, Jan-Peter**
**68535 Edingen-Neckarhausen (DE)**

(56) Entgegenhaltungen:
**EP-A- 0 839 526      WO-A-03/051338**
**WO-A-03/072084      US-A1- 2001 010 825**
**US-B2- 6 696 085**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft pharmazeutische Formulierungen in Form von Agglomeraten für die Herstellung von schnell zerfallenden Tabletten, enthaltend Zucker oder Zuckeralkohole, quervernetztes Polyvinylpyrrolidon und wasserunlösliche Polymere.

Im Mund schnell zerfallende und/oder sich schnell auflösende Tabletten gewinnen für die orale Applikation von Arzneistoffen immer größere Bedeutung. Solche Tabletten müssen innerhalb kurzer Zeit, am besten innerhalb von 30 Sekunden in der Mundhöhle zerfallen, angenehm schmecken und dürfen kein sandiges Gefühl hinterlassen. Ferner sollen sie einfach herstellbar sein, wobei die Direkttablettierung erhebliche Vorteile gegenüber der Feuchtgranulation bietet, und eine hohe mechanische Festigkeit besitzen, damit sie Verpackungsprozeduren, Transporte und auch das Herausdrücken aus Verpackungen unbeschadet überstehen.

Die bisher beschriebenen Produkte und Verfahren erfüllen diese Anforderungen nicht oder nur sehr unzureichend.

Schnell zerfallende Tabletten bestehen häufig aus Zucker und Zuckeralkoholen, Brausesystemen, mikrokristalliner Cellulose und anderen nicht wasserlöslichen Füllstoffen Calciumhydrogenphosphat, Cellulose-Derivaten, Maisstärke, oder Polypeptiden. Weiterhin kommen wasserlösliche Polymere, übliche Sprengmittel (quervernetztes PVP, Na- und Ca-Salz der quervernetzten Carboxymethylcellulose, Na-Salz der Carboxymethylstärke, niedrigsubstituierte Hydroxypropylcellulose L-HPC) und im wesentlichen anorganische wasserunlösliche Bestandteile (Kieselsäuren, Silikate, anorganische Pigmente) zum Einsatz. Weiterhin können die Tabletten auch Tenside enthalten.

In der WO 2003/051338 ist eine direkttablettierbare und gut verpressbare Hilfsstoffformulierung, welche Mannit und Sorbit enthält, beschrieben. Zunächst wird durch Lösen von Mannit und Sorbit in Wasser und anschließender Sprühtrocknung (gewöhnliche Sprühtrocknung und SBD-Verfahren) eine Hilfsstoffvormischung hergestellt. Dieser coprozessierten Mischung kann zusätzlich Mannit zugesetzt werden. Tabletten, welche zusätzlich Sprengmittel, Trennmittel, Pigment und einen Wirkstoff enthalten, sollen innerhalb von 60 Sekunden in der Mundhöhle zerfallen.

In der US 2002/071864 A1 wird eine Tablette beschrieben, welche innerhalb von 60 Sekunden in der Mundhöhle zerfällt, und hauptsächlich aus einer physikalischen Mischung von sprühgetrocknetem Mannit und einem grobkörnigen quervernetztem Polyvinylpyrrolidon sowie einer begrenzten Auswahl an Wirkstoffen formuliert ist. Diese Tabletten besitzen eine Bruchfestigkeit von ca. 40N und erzeugen ein unangenehmes, sandiges Mundgefühl.

Gemäß der US 6,696,085 B2 soll ein Methacrylsäure-Copolymer Typ C als Zerfallsmittel eingesetzt werden. Das Methacrylsäure-Copolymer Typ C ist ein magensaftresistentes Polymer, welches im sauren pH-Bereich nicht löslich ist, im pH-Bereich von 7, wie er in der Mundhöhle vorliegt, aber wasserlöslich ist. Die Tabletten weisen neben einer niedrigen Bruchfestigkeit (<20N) eine hohe Friabilität (>7%) auf und beinhalten einen hohen Anteil im Bereich von 15Gew.-% eines grobkörnigen Sprengmittels. Sie besitzen folglich eine niedrige mechanische Festigkeit und erzeugen aufgrund des hohen Anteils an grobkörnigem Sprengmittel ein unangenehmes, sandiges Mundgefühl.

EP 0839526 A2 beschreibt eine pharmazeutische Darreichungsform bestehend aus einem Wirkstoff, Erythrit, kristalliner Cellulose und einem Sprengmittel. Weiterhin wird Mannit eingearbeitet und als Sprengmittel quervernetztes Polyvinylpyrrolidon verwendet, sodass eine physikalische Mischung entsteht. Die Tabletten sollen innerhalb von 60 Sekunden in der Mundhöhle zerfallen.

In der Anmeldung JP 2004-265216 wird eine im Mund innerhalb von 60 Sekunden zerfallende Tablette, bestehend aus einem Wirkstoff, einem wasserlöslichen Polyvinylalkohol-Polyethylenglykol-Copolymer, Zucker/Zuckeralkohol (Mannit) und Sprengmittel, beschrieben.

Aufgabe der vorliegenden Erfindung war es, im Mund schnell zerfallende Tabletten zu finden, die ein angenehmes Mundgefühl hinterlassen und mechanisch sehr stabil sind.

Demgemäß wurde eine pharmazeutische Zubereitung für die Herstellung von im Mund schnell zerfallenden Tabletten gefunden, welche aus Agglomeraten enthaltend

    a) 60 - 97 Gew.-% mindestens eines Zuckers oder Zuckeralkohols oder Mischungen davon,
    b) 1 - 25 Gew.-% eines quervernetzten Polyvinylpyrrolidons,
    c) 1 - 15 Gew.-% von wasserunlöslichen Polymeren,
    d) 0 - 15 Gew.-% wasserlöslichen Polymeren, und
    e) 0 - 15 Gew.-% weiterer pharmazeutisch üblichen Hilfsstoffen,

besteht, wobei die Summe der Komponenten a) bis e) 100 Gew.-% beträgt.

Weiterhin wurde ein Verfahren zur Herstellung solcher Agglomerate gefunden.

Weiterhin wurden im Mund schnell zerfallende Tabletten, enthaltend solche Zubereitungen, gefunden. Die Tabletten zerfallen im Mund oder in wässrigem Milieu innerhalb von 40 Sekunden, bevorzugt innerhalb von 30 Sekunden, besonders bevorzugt innerhalb von 20 Sekunden.

Die pharmazeutischen Zubereitungen enthalten als Komponente a) 60 bis 97 Gew.-%, bevorzugt 70 bis 95 Gew.-%, besonders bevorzugt 75 bis 93 Gew.-% eines Zuckers, Zuckeralkohols oder Mischungen davon. Als Zucker oder Zuckeralkohole eignen sich Trehalose, Mannit, Erythrit, Isomalt, Maltit, Lactit, Xylit, Sorbit. Die Zucker- oder Zuckeralkoholkomponenten sind bevorzugt feinteilig, mit mittleren Teilchengrößen von 5 bis 100 $\mu$m. Gewünschtenfalls können die Teilchengrößen durch Mahlen eingestellt werden.

Bevorzugt werden Mannit, Erythrit oder Mischungen davon eingesetzt.

Als Komponente b) werden quervernetzte Polyvinylpyrrolidone in Mengen von 1 bis 25 Gew.-%, bevorzugt, 2 bis 15 Gew.-%, besonders bevorzugt 3 bis 10 Gew.-%, eingesetzt. Solche quervernetzten Polyvinylpyrrolidone sind wasserunlöslich, aber nicht filmbildend. Das quervernetzte Polyvinylpyrrolidon kann eine mittlere Teilchengröße von 2 bis 60 $\mu$m, bevorzugt kleiner 50 $\mu$m, besonders bevorzugt kleiner 30 $\mu$m aufweisen. Ganz besonders bevorzugt sind quervernetzte Polyvinylpyrrolidone mit einer Hydratationskapazität von größer 6,5 g/g. Hierbei erfolgt die Bestimmung der Hydratationskapazität nach folgender Methode:

2 g Polymer werden in ein Zentrifugenglas eingewogen und mit 40ml Wasser 15 Minuten ausquellen lassen. Anschließend wird 15 Minuten bei 2000U/Min zentrifugiert und die überstehende Flüssigkeit so vollständig wie möglich abgegossen.

$$\text{Hydratationskapazität} = \frac{\text{Auswaage-Tara}}{\text{Einwaage}}$$

Die hohe Hydratationskapazität des quervernetzten Polyvinylpyrrolidons führt in der Formulierung zu einem sehr schnellen Zerfall und ergibt ein besonders weiches Mundgefühl.

Als Komponente c) werden wasserunlösliche Polymere eingesetzt in Mengen von 1 bis 15 Gew.-%, bevorzugt 1 bis 10 Gew.-%, eingesetzt. Dabei handelt es sich um Polymere , . Bevorzugt sind Polymere, die im pH-Bereich von 1 bis 14 unlöslich sind, also eine bei jedem pH-Wert pH-unabhängige Wasserunlöslichkeit aufweisen. Weiterhin eignen sich aber auch Polymere, die bei jedem pH-Wert im pH-Bereich von 6 bis 14 wasserunlöslich sind.

Die Polymere sollen filmbildende Polymere sein. Filmbildend bedeutet in diesem Zusammenhang, dass die Polymere in wässriger Dispersion eine Mindestfilmbildetemperatur von -20 bis +150 °C, bevorzugt 0 bis 100 °C aufweisen.

Geeignete Polymere sind Polyvinylacetat, Ethylcellulose, Methylmethacrylat-Ethylacrylat-Copolymere, Ethylacrylat-Methylmethacrylat-Trimethylammoniumethylmethacrylat-Terpolymere. Butylmethacrylat-Methylmethacrylat-Dimethylaminoethylmethacrylat-Terpolymere

Die Acrylat-Methacrylat-Copolymere sind näher beschrieben in der Europäischen Pharmacopoeia als Polyacrylate Dispersion 30%, in der USP als Ammonio Methacrylate Copolymer und in JPE als Aminoalkyl-Methacrylate Copolymer E. Als bevorzugte Komponente c) kommt Polyvinylacetat zum Einsatz. Dieses kann als wässrige Dispersion mit Feststoffgehalten von 10 bis 45 Gew.-% eingesetzt werden. Bevorzugt ist zudem Polyvinylacetat mit einem Molekulargewicht zwischen 100:000 und 1.000.000 Dalton besonders bevorzugt zwischen 200.000 und 800.000 Dalton.

Weiterhin können die Formulierungen als Komponenten d) wasserlösliche Polymere in Mengen von 0 bis 15 Gew.-% enthalten. Geeignete wasserlösliche Polymere sind beispielsweise Polyvinylpyrrolidone, Vinylpyrrolidon-Vinylacetat-Copolymere, Polyvinylalkohole, Polyvinylalkohol-Polyethylenglykol-Pfropfcopolymere, Polyethylenglykole, Ethylenglykol-Propylenglykol-Blockcopolymere, Hydroxypropylmethylcellulose, Hydroxypropylcellulose, Hydroxyethylcellulose, Carragenane, Pektine, Xanthane, Alginate.

Gewünschtenfalls können durch Zusatz von pharmazeutisch üblichen Hilfsstoffen (Komponenten e)) in Mengen von 0 bis 15 Gew.-%, beispielsweise wie Säuerungsmitteln, Puffersubstanzen, Süßstoffen, Aromen, Geschmacksverstärkern und Farbstoffen Geschmack und Aussehen der aus den Formulierungen erhaltenen Tabletten weiter verbessert werden. Folgende Stoffe sind hierbei besonders geeignet: Citronensäure, Weinsäure, Ascorbinsäure, Natriumdihydrogenphosphat, Cyclamat, Saccharin-Na, Aspartam, Menthol, Pfefferminzaroma, Fruchtaromen, Vanillearoma, Glutamat, Riboflavin, Betacarotin, wasserlösliche Farbstoffe, feinteilige Farblacke.

Durch Zusatz von Verdickungsmitteln wie hochmolekularen Polysacchariden kann das Mundgefühl durch Erhöhung der Weichheit und des Volumengefühls zusätzlich verbessert werden.

Weiterhin können als Komponenten e) auch Tenside zugegeben werden. Als Tenside eignen sich beispielsweise Natriumlaurylsulfat, Dioctylsulfosuccinat, alkoxilierte Sorbitanester wie Polysorbat 80, polyalkoxilierte Derivate von Rizinusöl oder hydriertem Rizinusöl, beispielsweise Cremophor® RH 40, alkoxilierte Fettsäuren, alkoxilierte Hydroxyfettsäuren, alkoxilierte Fettalkohole, Alkalisalze von Fettsäuren und Lecithine. Weiterhin können zur weiteren Verbesserung des Zerfalls auch feinteilige Pigmente zugegeben werden, weil sie die inneren Grenzflächen erhöhen und somit Wasser schneller in die Tablette eindringen kann. Diese Pigmente wie Eisenoxide, Titandioxid, kolloidale oder gefällte Kieselsäure, Calciumcarbonate, Calciumphosphate müssen natürlich sehr feinteilig sein, ansonsten entsteht wiederum ein körniger Geschmack.

Die Herstellung der erfindungsgemäßen Formulierungen kann durch Aufbau-Agglomeration in Mischern, Wirbelschichtgeräten oder Sprühtürmen erfolgen. Dabei werden feste Ausgangsmaterialien und Granulierflüssigkeit zunächst miteinander vermischt und das feuchte Mischgut anschliessend getrocknet. Gemäß der vorliegenden Erfindung wird als Granulierflüssigkeit eine wässrige Dispersion der Komponente c), des wasserunlöslichen Polymers, eingesetzt.

Bei der Agglomeration in der Wirbelschicht wird eine wässrige Dispersion des wasserunlöslichen Polymers auf eine wirbelnde Mischung aus Zucker oder Zuckeralkohol und quervernetztem PVP gesprüht, wodurch die feinen Teilchen agglomerieren. Die Zulufttemperaturen betragen 30 bis 100°C, die Ablufttemperaturen 20 bis 70°C.

Bei der Herstellung in Sprühtürmen wird vorzugsweise die sogenannte FSD- oder SBD-Technologie (FSD: Fluidized spray drying; SBD: Spray bed drying) eingesetzt. Hierbei wird eine Lösung des Zuckers oder Zuckeralkohols in Wasser zunächst sprühgetrocknet, im unteren Teil des Sprühtrockners oder in einem angeschlossenen Wirbelbett erfolgt die Zugabe von quervernetztem PVP und die Eindüsung einer wässrigen Dispersion des wasserunlöslichen Polymeren, wodurch die Teilchen agglomerieren. Feine Teilchen können ferner nochmals vor die Sprühdüse der Zucker oder Zuckeralkohollösung geblasen werden und zusätzlich agglomeriert werden. Es ist im Sprühturm, FSD oder SBD auch eine Prozeßführung ausgehend von der kristallinen Form des Zuckers oder Zuckeralkohols möglich. Dabei wird der kristalline Zucker oder Zuckeralkohol am Kopf des Sprühturms oder in den Feingut-Recyclingstrom zugegeben. Durch das Versprühen einer wässrigen Dispersion des wasserunlöslichen Polymeren wird dieser kristalline Feststoff im Turm agglomeriert.

Für den Agglomerationsprozess kann es sich als günstig erweisen, einen mehrstufigen Sprühprozess zu fahren. Zu Anfang wird die Sprührate niedrig gehalten, um ein Überfeuchten der Produktvorlage und damit deren Verkleben zu verhindern. Mit zunehmender Prozessdauer kann die Sprührate erhöht und damit die Agglomerationstendenz erhöht werden. Es ist ebenfalls möglich die Zuluftmenge und/oder -temperatur in entsprechender Weise während des Prozesses anzupassen. Besonders während der Trocknungsphase ist es vorteilhaft, die Zuluftmenge zu reduzieren und damit einem Abrieb der Agglomerate durch eine hohe mechanischen Belastung vorzubeugen.

Als weitere Anpassungsparameter für die Agglomeratgröße kann die Feinheit des Sprühtröpfchens der Bindemittellösung bzw. -dispersion (einstellbar über den Zerstäubungsgasdruck), die Düsengeometrie und Abstand der Düse zum Produktbett angesehen werden. Je feiner und einheitlicher gesprüht wird, desto feiner und einheitlicher resultieren die Agglomerate. Je weiter die Düse vom Produktbett entfernt ist, desto schlechter ist das Agglomerationsverhalten.

Weiterhin können die Agglomerate auch in einem Mischer durch eine kontinuierlich geführte Mischaggregation erfolgen. Eine solche kontinuierlich geführte Form der Mischaggregation ist die sogenannte "Schugi-Granulation". Dabei werden in einem kontinuierlich arbeitenden vertikal angeordneten Hochgeschwindigkeitsmischer feste Ausgangsmaterialien und die das wasserunlösliche Polymer enthaltende Granulierflüssigkeit intensiv miteinander vermischt (siehe auch M. Bohnet, "Mechanische Verfahrenstechnik", Wiley VCH Verlag, Weinheim 2004, S. 198 ff.)

Gemäß einer besonderen Ausführungsform wird das quervenetzte PVP in der wässrigen Dispersion des wasserunlöslichen Polymers suspendiert.

Die so erhaltenen Agglomerate weisen eine mittlere Teilchengröße von 100 - 600 $\mu$m, bevorzugt 120 - 500 $\mu$m und besonders bevorzugt von 140 - 400 $\mu$m auf.

Das wasserunlösliche, filmbildende Polymer dient dabei als Agglomerationsmittel, um die feinen Zucker oder Zuckeralkoholkristalle und die Teilchen von quervernetztem PVP zu agglomerieren.

Die erfindungsgemäßen Formulierungen können vorteilhaft auch für die Herstellung von Tabletten verwendet werden, die vor der Anwendung in einem Glas Wasser zerfallen gelassen werden. Auch die Herstellung von Tabletten, die intakt geschluckt werden, ist natürlich möglich.

Für die Herstellung von Tabletten können die üblichen Verfahren verwendet werden, wobei die Direkttablettierung und die Walzenkompaktierung besondere Vorteile bieten. Aufgrund der besonderen Eigenschaften der erfindungsgemäßen Formulierungen werden in der Regel nur Wirkstoff, erfindungsgemäße Formulierung und ein Schmiermittel benötigt. Die Tablettenrezeptur ist somit sehr einfach, sehr reproduzierbar und das Verfahren leicht zu validieren.

Überraschenderweise wurde gefunden, dass ein wasserunlösliches filmbildendes Polymer den Zerfall von Tabletten erheblich beschleunigt. Dies ist umso überraschender, da solche Polymere in der Regel für die Herstellung von retardierten Arzneiformen eingesetzt werden, die innerhalb mehrerer Stunden nicht zerfallen. Die Zerfallszeiten unter Verwendung von Polyvinylacetat als wasserunlöslichem Polymer sind erheblich kürzer als bei wasserlöslichen Polymeren.

Ferner besitzen die erfindungsgemäßen Zubereitungen außergewöhnlich gute Fließfähigkeiten und Verpressbarkeiten, die zu mechanisch sehr stabilen Tabletten führen.

Die Bruchfestigkeit der mit Hilfe der erfindungsgemäßen pharmazeutischen Formulierungen erzeugten Tabletten beträgt > 50 N. Häufig liegen die Bruchfestigkeiten oberhalb von 80 N, selbst unter Verwendung schwer pressbarer Wirkstoffe. Die Friabilitäten betragen < 0,2 %. Beschädigungen beim üblichen Tablettenhandling treten somit nicht auf.

Aufgrund des feinen quervernetzten Polyvinylpyrrolidons zeigen die Tabletten nahezu keine Veränderungen der Tablettenoberfläche bei feuchter Lagerung. Im Gegensatz zu grobem quervernetzten Polyvinylpyrrolidon findet sich keine Pickelbildung durch stark aufgequollene Teilchen. Die erfindungsgemäßen Formulierungen sind damit bei Lagerung sehr stabil und behalten ihr ansprechendes Aussehen.

Beispiele

Beispiele A - H zeigen den zerfallsfördernden Effekt von Polyvinylacetat als wasserunlösliches Polymer gegenüber wasserlöslichen Polymeren.

Zunächst wurden Agglomerate in der Wirbelschicht hergestellt: Zucker/Zuckeralkohol und quervernetztes PVP wurden vorgelegt und mit wässrigen Bindemittellösungen/- dispersionen, die in den Wirbelschichtgranulator ( Firma Glatt,GPCG 3.1) mittels Topspray-Verfahren eingesprüht wurden, agglomeriert. Erythrit wurde aufgrund seiner groben Körnung zunächst zu einem feinem Pulver zerkleinert.

Die Herstellung erfolgte durch einen zweistufigen Agglomerationsprozess, wobei zunächst eine geringere Sprührate gewählt wurde und dann die Sprührate erhöht wurde.

Folgende Herstellungsbedingungen wurden in einem zweistufigen Agglomerationsprozess angewendet:

| | |
|---|---|
| Ansatzgröße: | 0,6 kg |
| Konzentration der Bindemittellösung/-dispersion: | 10 Gew.-% |
| Zulufttemperatur: | 55 °C |
| Ablufttemperatur zu Beginn: | 35 °C |
| Ablufttemperatur nach Umstellen der Sprührate: | 25 °C |
| Sprührate zu Beginn: | 7,5 g/min |
| Sprührate nach Umstellen: | 20 g/min |

Tabelle 1: Rezepturzusammensetzung der Agglomerate A bis H in Gew.-%

| | A | B | C | D | E | F | G | H |
|---|---|---|---|---|---|---|---|---|
| Laktose (Granulac 230) | 93 | 93 | - | - | - | - | - | - |
| Mannit (Pearlitol 25 C) | - | - | 90 | 90 | 90 | 90 | 45 | 45 |
| Erythrit (Eridex 16952) | - | - | - | - | - | - | 45 | 45 |
| quervernetztes PVP (Kollidon CL) | 3,5 | 3,5 | 5,0 | 5,0 | 5,0 | 5,0 | 5,0 | 5,0 |
| PVP (Kollidon 30) | 3,5 | - | 5,0 | - | - | - | 5,0 | - |
| Polyvinylalkohol / Polyethylenglykol Block-Copolymer (Kollicoat IR) | - | - | - | 5,0 | - | - | - | - |
| Methacrylsäure / Ethylacrylat Copolymer (Kollicoat MAE 100 P) | - | - | - | - | 5,0 | - | - | - |
| Polyvinylacetat | - | 3,5 | - | - | - | 5,0 | - | 5,0 |

Die so hergestellten Agglomerate wurden mit 0,5 bis 1,0 Gew.-% Schmiermittel (Mg-Stearat) in einem Turbulamischer für 5 min gemischt. Anschließend wurden diese Mischungen auf einer voll instrumentierten Rundläufertablettenpresse (Korsch PH 100/6) bei einer Umdrehungsgeschwindigkeit von 30 Upm tablettiert. Die Rundläufertablettenpresse war mit 6 Stempeln (10 mm, biplan, facettiert) ausgestattet. Das Tablettengewicht wurde auf 300 mg eingestellt. Zunächst erfolgte eine Tablettierung bei einer Presskraft von 18 kN (die Tabletten wurden je nach Verpressbarkeit des Pulvers unterschiedlich hart), anschließend wurde die Presskraft jeweils so eingestellt, dass die Bruchfestigkeit der Tabletten 60 N betrug.

Die Tabletten wurden hinsichtlich Bruchfestigkeit (Tablettentester HT-TMB-CI-12 F, Fa. Kraemer), Friabilität (Roche-Friabilator, Erweka) und Zerfallszeit in Phosphatpuffer pH 7,2 (Zerfallstester ZT 74, Erweka) untersucht. Die Zahlenangaben links des Schrägstriches beziehen sich auf die Tabletten, die mit einer Presskraft von 18 kN erhalten wurden.

Tabelle 2: Tabletteneigenschaften Formulierungen A bis H

| | Bruchfestigkeit [N] | Friabilität [%] | Zerfallszeit [s] |
|---|---|---|---|
| A | 180 / 60 | 0,10 / 0,15 | 120 / 75 |
| B | 180 / 60 | 0,05 / 0,15 | 45 / 20* |
| C | 200 / 60 | 0,15 / 0,20 | 180 / 120 |
| D | 250 / 60 | 0,15 / 0,25 | 210 / 150 |

(fortgesetzt)

|   | Bruchfestigkeit [N] | Friabilität [%] | Zerfallszeit [s] |
|---|---|---|---|
| E | 220 / 60 | 0,10 / 0,25 | 240 / 180 |
| F | 200 / 60 | 0,02 / 0,15 | 60 / 20* |
| G | 200 / 60 | 0,20 / 0,30 | 180 / 120 |
| H | 200 / 60 | 0,10 / 0,25 | 80 / 30 |
| * Bestimmung von Zerfallszeiten < 20 s ist aus Gründen der Endpunktserkennung nicht möglich. | | | |

Beispiele J bis M zeigen die Eignung eines schnell zerfallenden Hilfsstoffes in einer Wirkstoffformulierung.
Der schnell zerfallende Hilfsstoff wird durch Agglomeration in der Wirbelschicht von Mannit (90 Gew.-%) und quervernetztem PVP (5 Gew.-%) mit Polyvinylacetat (5 Gew.-%) hergestellt. Das so hergestellte Direkttablettierungsmittel wurde mit Wirkstoff und 0,5 bis 1,0 Gew.-% Schmiermittel (Mg-Stearat) abgemischt und anschließend auf einer Rundläufertablettenpresse (Korsch PH 100/6) zu Tabletten mit 60 N Bruchfestigkeit verpresst.

Tabelle 3: Wirkstoff, Wirkstoffmenge, Tablettengewicht und Durchmesser der Wirkstoffformulierungen J bis M

|   | Wirkstoff | Wirkstoffmenge | Tablettengewicht | Durchmesser |
|---|---|---|---|---|
| J | Loratadin | 10 mg | 250 mg | 8mm |
| K | Loperamid-HCl | 2 mg | 100 mg | 6 mm |
| L | Cetirizin-2HCl | 10 mg | 280 mg | 10 mm |
| M | Lorazepam | 2 mg | 120 mg | 7 mm |

Die Tabletten wurden hinsichtlich Bruchfestigkeit (Tablettentester HT-TMB-Cl-12 F, Fa. Kraemer), Friabilität (Roche-Friabilator, Erweka) und Zerfallszeit in Phosphatpuffer pH 7,2 (Zerfallstester ZT 74, Erweka) untersucht.

Tabelle 4: Tabletteneigenschaften Formulierungen J bis M

|   | Bruchfestigkeit [N] | Friabilität [%] | Zerfallszeit [s] |
|---|---|---|---|
| J | 60 | < 0,20 | 30 |
| K | 60 | < 0,20 | 20* |
| L | 60 | < 0,20 | 25 |
| M | 60 | < 0,20 | 20* |
| * Bestimmung von Zerfallszeiten < 20 s ist aus Gründen der Endpunktserkennung nicht möglich. | | | |

**Patentansprüche**

1.  Pharmazeutische Formulierung in Form von Agglomeraten enthaltend

    a) 60 - 97 Gew.-% Zucker oder Zuckeralkohole,
    b) 1 - 25 Gew.-% quervernetztes Polyvinylpyrrolidon,
    c) 1 - 15 Gew.-% wasserunlösliche, filmbildende Polymere
    d) 0 - 15 Gew.-% wasserlösliche Polymere, und
    e) 0 - 15 Gew.-% weitere pharmazeutisch übliche Hilfsstoffe
    wobei die Summe der Komponenten a) bis e) 100 Gew.-% beträgt.

2.  Formulierung nach Anspruch 1 **dadurch gekennzeichnet, dass** die mittlere Teilchengröße der Agglomerate 100 $\mu$m bis 600 $\mu$m beträgt.

3.  Formulierung nach Anspruch 1 oder 2, enthaltend als Zuckeralkohol Mannit oder Erythrit oder Mischungen davon.

**4.** Formulierung nach einem der Ansprüche 1 bis 3, enthaltend ein quervenetztes Polyvinylpyrrolidon mit einer mittleren Teilchengröße kleiner 50 μm.

**5.** Formulierung nach einem der Ansprüche 1 bis 4, enthaltend ein quervernetztes Polyvinylpyrrolidon mit einer Hydratationskapazität größer 6,5 g/g.

**6.** Formulierung nach einem der Ansprüche 1 bis 5 , wobei als wasserunlösliches filmbildendes Polymer Polyvinylacetat verwendet wird.

**7.** Formulierung nach einem der Ansprüche 1 bis 6, wobei das wasserunlösliche filmbildende Polymer Polyvinylacetat in Form einer wässrigen Dispersion eingesetzt wird.

**8.** Formulierung nach einem der Ansprüche 1 bis 7 , wobei als wasserlösliches Polymer Polyvinylpyrrolidon verwendet wird.

**9.** Formulierung nach einem der Ansprüche 1 bis 8, wobei als weitere pharmazeutisch übliche Stoffe Säuerungsmittel, Süßstoffe, Aromen, Geschmacksverstärker, Farbstoffe, Verdickungsmittel, Tenside und feinteilige Pigmente verwendet werden.

**10.** Formulierung nach einem der Ansprüche 1 bis 9, enthaltend Agglomerate aus

　　a) 70 - 95 Gew.-% Zucker oder Zuckeralkoholen
　　b) 2 - 15 Gew.-% quervernetztes Polyvinylpyrrolidon,
　　c) 1 - 10 Gew.-% wasserunlösliche, filmbildende Polymere
　　d) 0 - 2 Gew.-% wasserlösliches Polyvinylpyrrolidon, und
　　e) 0 - 15 Gew.-% weitere pharmazeutisch übliche Hilfsstoffe.

**11.** Formulierung nach einem der Ansprüche 1 bis 10, enthaltend Agglomerate aus

　　a) 75 - 95 Gew.-% Mannit oder Erythrit oder einer Mischung davon,
　　b) 3 - 10 Gew.-% quervernetztes Polyvinylpyrrolidon,
　　c) 1- 10 Gew.-% Polyvinylacetat,
　　d) 0 - 2 Gew.-% wasserlösliches Polyvinylpyrrolidon, und
　　e) 0 - 15 Gew.-% weitere pharmazeutisch übliche Hilfsstoffe

**12.** Tabletten erhalten unter Verwendung einer pharmazeutischen Formulierung nach einem der Ansprüche 1 bis 9, wobei die Tabletten in wässrigem Milieu Zerfallszeiten von kleiner 30 Sekunden aufweisen.

**13.** Tabletten nach Anspruch 10, wobei die Tabletten eine Bruchfestigkeit größer 50 N aufweisen.

**14.** Tabletten nach Anspruch 10 oder 11, enthaltend 20 bis 99 Gew.-%, bezogen auf das gesamte Tablettengewicht, einer pharmazeutischen Formulierung gemäß einem der Ansprüche 1 bis 9.

**15.** Tabletten nach einem der Ansprüche 10 bis 12, enthaltend weitere Hilfsstoffe.

**16.** Verfahren zur Herstellung einer pharmazeutischen Formulierung gemäß einem der Ansprüche 1 bis 9, **dadurch gekennzeichnet, dass** feinteilige Zucker- oder Zuckeralkoholteilchen und quervernetztes Polyvinylpyrrolidon mit einer wässrigen Dispersion des wasserunlöslichen Polymers agglomeriert werden.

**17.** Verfahren nach Anspruch 16, **dadurch gekennzeichnet, dass** feinteilige Zucker- oder Zuckeralkoholteilchen mit einer wässrigen Dispersion des wasserunlöslichen Polymers, die zusätzlich quervernetztes Polyvinylpyrrolidon suspendiert enthält, agglomeriert werden.

**18.** Verfahren nach Anspruch 16 oder 17 , **dadurch gekennzeichnet, dass** die Agglomeration in einem Wirbelschichtgranulator, einem Mischer oder einem Sprühturm erfolgt.

**Claims**

1.  A pharmaceutical formulation in the form of agglomerates comprising

    a) 60-97% by weight of sugar or sugar alcohols,
    b) 1-25% by weight of crosslinked polyvinylpyrrolidone,
    c) 1-15% by weight of water-insoluble, film-forming polymers,
    d) 0-15% by weight of water-soluble polymers and
    e) 0-15% by weight of further pharmaceutically customary excipients,

    the sum of the components a) to e) being 100% by weight.

2.  The formulation according to claim 1, wherein the mean particle size of the agglomerates is from 100 $\mu$m to 600 $\mu$m.

3.  The formulation according to claim 1 or 2, comprising mannitol or erythritol or mixtures thereof as the sugar alcohol.

4.  The formulation according to any of claims 1 to 3, comprising a crosslinked polyvinylpyrrolidone having a mean particle size of less than 50 $\mu$m.

5.  The formulation according to any of claims 1 to 4, comprising a crosslinked polyvinylpyrrolidone having a hydration capacity greater than 6.5 g/g.

6.  The formulation according to any of claims 1 to 5, polyvinyl acetate being used as the water-insoluble film-forming polymer.

7.  The formulation according to any of claims 1 to 6, polyvinyl acetate in the form of an aqueous dispersion being used as the water-insoluble film-forming polymer.

8.  The formulation according to any of claims 1 to 7, polyvinylpyrrolidone being used as the water-soluble polymer.

9.  The formulation according to any of claims 1 to 8, acidifying agents, sweeteners, flavors, flavor enhancers, colorants, thickeners, surfactants and finely divided pigments being used as further pharmaceutically customary substances.

10. The formulation according to any of claims 1 to 9, comprising agglomerates of

    a) 70-95% by weight of sugar or sugar alcohols,
    b) 2-15% by weight of crosslinked polyvinylpyrrolidone,
    c) 1-10% by weight of water-insoluble, film-forming polymers,
    d) 0-2% by weight of water-soluble polyvinylpyrrolidone and
    e) 0-15% by weight of further pharmaceutically customary excipients.

11. The formulation according to any of claims 1 to 10, comprising agglomerates of

    a) 75-95% by weight of mannitol or erythritol or a mixture thereof,
    b) 3-10% by weight of crosslinked polyvinylpyrrolidone,
    c) 1-10% by weight of polyvinyl acetate,
    d) 0-2% by weight of water-soluble polyvinylpyrrolidone and
    e) 0-15% by weight of further pharmaceutically customary excipients.

12. A tablet obtained using a pharmaceutical formulation according to any of claims 1 to 9, the tablet having a disintegration time of less than 30 seconds in an aqueous medium.

13. The tablet according to claim 10, the tablet having a hardness greater than 50 N.

14. The tablet according to claim 10 or 11, comprising from 20 to 99% by weight, based on the total tablet weight, of a pharmaceutical formulation according to any of claims 1 to 9.

15. The tablet according to any of claims 10 to 12, comprising further excipients.

**16.** A process for the preparation of a pharmaceutical formulation according to any of claims 1 to 9, wherein finely divided sugar or sugar alcohol particles and crosslinked polyvinylpyrrolidone are agglomerated with an aqueous dispersion of the water-insoluble polymer.

**17.** The process according to claim 16, wherein finely divided sugar or sugar alcohol particles are agglomerated with an aqueous dispersion of the water-insoluble polymer which additionally comprises suspended crosslinked polyvinylpyrrolidone.

**18.** The process according to claim 16 or 17, wherein the agglomeration is effected in a fluidized-bed granulator, a mixer or a spray tower.

**Revendications**

**1.** Formulation pharmaceutique sous forme d'agglomérats contenant

a) 60-97% en poids de sucre ou d'alcools de sucre
b) 1-25% en poids de polyvinylpyrrolidone réticulée,
c) 1-15% en poids de polymères filmogènes insolubles dans l'eau
d) 0-15% en poids de polymères solubles dans l'eau, et
e) 0-15% en poids d'autres adjuvants pharmaceutiquement usuels,
la somme des composants a) à e) valant 100% en poids.

**2.** Formulation selon la revendication 1, **caractérisée en ce que** la grosseur moyenne des agglomérats est de 100 $\mu$m à 600 $\mu$m.

**3.** Formulation selon la revendication 1 ou 2, contenant, comme alcool de sucre, du mannitol, de l'érythritol ou des mélanges de ceux-ci.

**4.** Formulation selon l'une quelconque des revendications 1 à 3, contenant une polyvinylpyrrolidone réticulée présentant une grosseur moyenne de particule inférieure à 50 $\mu$m.

**5.** Formulation selon l'une quelconque des revendications 1 à 4, contenant une polyvinylpyrrolidone réticulée présentant une capacité d'hydratation supérieure à 6,5 g/g.

**6.** Formulation selon l'une quelconque des revendications 1 à 5, du poly(acétate de vinyle) étant utilisé comme polymère filmogène insoluble dans l'eau.

**7.** Formulation selon l'une quelconque des revendications 1 à 6, le poly(acétate de vinyle) sous forme d'une dispersion aqueuse étant utilisé comme polymère filmogène insoluble dans l'eau,.

**8.** Formulation selon l'une quelconque des revendications 1 à 7, de la polyvinylpyrrolidone étant utilisée comme polymère soluble dans l'eau.

**9.** Formulation selon l'une quelconque des revendications 1 à 8, des agents acidifiants, des édulcorants, des arômes, des exhausteurs de goût, des colorants, des épaississants, des agents tensioactifs et des pigments finement divisés étant utilisés comme autres substances pharmaceutiquement usuelles.

**10.** Formulation selon l'une quelconque des revendications 1 à 9, contenant des agglomérats constitués de

a) 70-95% en poids de sucre ou d'alcools de sucre
b) 2-15% en poids de polyvinylpyrrolidone réticulée,
c) 1-10% en poids de polymères filmogènes insolubles dans l'eau
d) 0-2% en poids de polyvinylpyrrolidone soluble dans l'eau, et
e) 0-15% en poids d'autres adjuvants pharmaceutiquement usuels.

**11.** Formulation selon l'une quelconque des revendications 1 à 10, contenant des agglomérats constitués de

a) 75-95% en poids de mannitol ou d'érythritol ou d'un mélange de ceux-ci,
b) 3-10% en poids de polyvinylpyrrolidone réticulée,
c) 1-10% en poids de poly(acétate de vinyle),
d) 0-2% en poids de polyvinylpyrrolidone soluble dans l'eau, et
e) 0-15% en poids d'autres adjuvants pharmaceutiquement usuels.

12. Comprimés obtenus par l'utilisation d'une formulation pharmaceutique selon l'une quelconque des revendications 1 à 9, les comprimés présentant des temps de décomposition en milieu aqueux inférieurs à 30 secondes.

13. Comprimés selon la revendication 10, les comprimés présentant une résistance à la rupture supérieure à 50 N.

14. Comprimés selon la revendication 10 ou 11, contenant 20 à 99% en poids, par rapport au poids total du comprimé, d'une formulation pharmaceutique selon l'une quelconque des revendications 1 à 9.

15. Comprimés selon l'une quelconque des revendications 10 à 12, contenant d'autres adjuvants

16. Procédé pour la préparation d'une formulation pharmaceutique selon l'une quelconque des revendications 1 à 9, **caractérisé en ce qu'**on agglomère des particules finement divisées de sucre ou d'alcool de sucre et de la polyvinylpyrrolidone réticulée avec une dispersion aqueuse du polymère insoluble dans l'eau.

17. Procédé selon la revendication 16, **caractérisé en ce qu'**on agglomère des particules finement divisées de sucre ou d'alcools de sucre avec une dispersion aqueuse du polymère insoluble dans l'eau, qui contient en outre de la polyvinylpyrrolidone réticulée en suspension.

18. Procédé selon la revendication 16 ou 17, **caractérisé en ce que** l'agglomération est réalisée dans un granulateur à lit fluidisé, un mélangeur ou une tour de pulvérisation.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2003051338 A
- US 20020071864 A1
- US 6696085 B2

- EP 0839526 A2
- JP 2004265216 A

**In der Beschreibung aufgeführte Nicht-Patentliteratur**

- **M. BOHNET.** Mechanische Verfahrenstechnik. Wiley VCH Verlag, 2004, 198 ff